# EUROPEAN PATENT APPLICATION

(11) **EP 0 834 302 A1**
(43) Date of publication of application: **08.04.1998**
(21) Application number: 97307502.1
(22) Date of filing: 25.09.1997
(51) Int. Cl.: A61K 7/00, A61K 7/48

(54) **Compositions comprising glutathione liposomes**

(30) Priority: 02.10.1996 GB 9620503
(71) Applicant: Boehringer Ingelheim Limited, Bracknell, Berkshire RG12 4YS (GB)
(72) Inventor: The designation of the inventor has not yet been filed
(74) Representative: Golding, Louise Ann

(57) **Abstract**

The invention provides compositions suitable for topical application comprising glutathione liposomes, together with at least one sunscreen agent and/or a delayed release carrier. Such compositions are capable of preventing or reducing the damaging effects of UV radiation on the skin.

## Description

The present invention relates to compositions comprising glutathione liposomes. In particular, the invention relates to the use of such compositions to help maintain or restore the skin's natural levels of glutathione, thereby preventing or reducing the harmful effects of UV radiation on the skin. Such compositions find particular use in combination with sunscreen agents in a sunscreen preparation.

The damaging effects of UV radiation on the skin are well documented. Short term effects of exposure to UV radiation include erythema (i.e. sunburn), whilst the long term effects of repeated exposure to UV radiation include skin cancers and premature ageing of the skin.

The formation of free radicals in the skin is believed to be a primary cause of the harmful effects of sunlight upon the skin. Whilst the skin itself contains antioxidants or free radical scavengers, the natural levels of these deplete rapidly on exposure to UV radiation, thus significantly reducing the body's own defence against damage from free radicals.

Amongst the free radical scavengers naturally present in the skin is glutathione. However, exposure to UV radiation is believed to be capable of reducing the skin's natural levels of glutathione in as little as 4 minutes, thereby reducing the body's natural defence against the harmful effects of free radicals. There thus exists a need for formulations which can be topically applied to the skin and which serve to replenish the skin's natural levels of glutathione, thereby preventing or reducing damage to the skin resulting from exposure to UV radiation.

Glutathione itself is unable to penetrate through the stratum corneum and applied topically to the skin in a simple oil-in-water emulsion or in the form of a gel preparation has been found to be ineffective in inhibiting UVB induced skin erythema (Int. J. Cos. Sci. 17:91-103, 1995).

It has now surprisingly been found that the use of glutathione in the form of a liposomal preparation provides effective levels of glutathione in the skin, thus serving to replenish and maintain the skin's natural levels of glutathione and thereby preventing or reducing the damaging effects of UV radiation on the skin.

Viewed from one aspect, the invention thus provides a composition suitable for topical application comprising glutathione liposomes, preferably in an amount of from 0.5 to 10% by weight, e.g. from 1 to 5% by weight, together with at least one sunscreen agent and/or a delayed release carrier.

As used herein, "delayed release carrier" is used to define any carrier suitable for topical application which serves to provide a delayed release of glutathione liposomes to the skin. In this way, a slow yet constant release of glutathione liposomes to the skin is provided over a prolonged period of time thereby ensuring that the level of glutathione in the skin is constantly replenished.

In a particularly preferred embodiment, the compositions in accordance with the invention comprise a delayed release carrier in addition to at least one sunscreen agent.

Release of the glutathione liposomes to the skin may be delayed by the use of materials known to prevent wash off of the formulation from the skin. This has the effect of prolonging the time the liposome is in contact with the skin whilst at the same time slowing or delaying its actual penetration into the stratum corneum. Such materials may be inherently water resistant, such as natural and synthetic waxes, microcrystalline hydrocarbon waxes, non-volatile silicones, silicone waxes or silicone based compounds. Alternatively, such materials may form a water insoluble polymeric film on the skin surface which prevents wash off. These materials act as a barrier to passage of the liposomes which will then slowly permeate the barrier and deliver a continuous amount of the liposome to the site of application.

Examples of silicone materials suitable for use as delayed release carriers include dimethicone, cetyl methicone, dimethiconol, trimethylsiloxysilicate, phenyltrimethicone, stearoxytrimethylsilane and copolymers thereof or any combination of such materials. Other agents suitable for use as delayed release carriers include natural waxes such as beeswax and candililla wax, synthetic waxes such as paraffin wax and hydrogenated vegetable oils, and water insoluble polymers such as modified acrylate copolymers and copolymers of polyvinylpyrrolidone (PVP), e.g. PVP/eicosene, PVP/hexadecene copolymer and tricontanyl PVP. Conveniently, the delayed release carrier will be present in an amount of from 0.5 to 20% by weight, preferably from 1.0 to 10% by weight.

The glutathione for use in accordance with the invention is preferably present in the form of reduced glutathione. Derivatives of glutathione, including its salts and esters, may however be used.

Glutathione liposomes suitable for use in accordance with the invention preferably comprise an aqueous solution containing reduced glutathione enclosed within a multilamellar liposomal matrix. Preferred liposomes are those comprising from 0.5 to 5.0% by weight, preferably from 1.5 to 2.3% by weight phospholipids which facilitate liposome penetration. Preferably, the liposome will range in size from 50 to 500nm, e.g. up to about 200nm. Suitable phospholipids for making the liposomes include phosphatidylcholine, phosphatidylethanolamine and phospatidylinositol which may be present in amounts of from 18 to 26% by weight, from 16 to 21% by weight and from 12 to 18% by weight respectively. The liposomal matrix may further contain preservatives to prevent spoilage by microorganisms. The final concentration of reduced glutathione present in the liposomal matrix is conveniently from 0.01 to 1.0% by weight, preferably 0.1% by weight.

In a preferred embodiment, the liposomes themselves may be formulated to provide a delayed release of glutathione. This may be achieved by incorporation of palmitoylcarnitine in the liposome membrane which not only provides a delayed release of glutathione but also has been found to contribute to the stability of the phospholipid membrane.

Liposomes for use in accordance with the invention can be prepared according to methods known in the art. For example, these can be prepared by dissolving at least one liposome forming compound in a suitable solvent.

As used herein, "sunscreen agent" is used to define any ingredient or ingredients conventionally used in sunscreen and/or sunblock preparations which are intended for topical application and which provide protection against UV radiation. The amount and type of sunscreen agent present in the compositions may readily be determined by those skilled in the art but typically will depend upon the desired SPF (Sun Protection Factor) of the product and its required photostability. Preferably, the sunscreen agent(s) will be sufficiently photostable to provide adequate protection during prolonged exposure to UV radiation without the need for regular re-application and, in addition, will preferably provide balanced UVA:UVB protection.

Sunscreen agents for use in accordance with the invention include both physical and chemical sunblocks. Chemical sunblocks are believed to act by absorbing UV radiation so that it cannot penetrate the skin, whereas physical sunblocks work by scattering and reflecting UV radiation. Chemical sunscreens absorb at different wavelengths. Those absorbing in the 290-320nm region are UVB screens and those absorbing in the 320-400nm region are UVA screens. Since the SPF of a sunscreen agent is measured in the UVB region, most commercially available sunscreen agents are UVB absorbers.

The sunscreen agents are conveniently present in the compositions of the invention in an amount of from 0.5 to 30% by weight, preferably from 5 to 25 % by weight, more preferably from 10 to 20 % by weight.

Examples of UVB absorbers suitable for use in accordance with the invention include 4-aminobenzoic acid (PABA) and its derivatives, cinnamates, salicylates and camphor derivatives, which may each be present in an amount of up to 10% by weight. Preferred UVB sunscreens include those selected from the group consisting of PABA in amounts of from 0.5 to 5% by weight, 2-ethylhexyl-4-dimethylaminobenzoate (octyldimethyl PABA) in an amount of from 0.5 to 8% by weight, 2-ethylhexyl-4-methoxycinnamate (octylmethoxycinnamate) or isopentyl-4-methoxycinnamate in amounts of from 0.5 to 10% by weight, 2-ethylhexylsalicylate (octylsalicylate) in an amount of from 0.5 to 5% by weight, homomenthylsalicylate (homosalate) in an amount of from 0.5 to 10% by weight and 3-(4'-methylbenzylidene)-d-1-camphor in an amount of from 0.5 to 6% by weight. Particularly preferred UVB absorbers include methylbenzylidene camphor commercially available as Eusolex 6300^{®} from Merck and octylmethoxycinnamate.

UVA absorbers suitable for use in accordance with the invention include the benzophenones and dibenzoylmethanes, e.g. 1-(4-t-butylphenyl)-3-(4-methoxyphenyl)propane-1,3-dione (butylmethoxydibenzoylmethane) in an amount of from 0.5 to 5% by weight, 2-hydroxy-4-methoxybenzophenone (oxybenzone, benzophenone-3) in an amount of from 0.5 to 10% by weight, or the water soluble benzophenone, 2-hydroxy-4-methoxybenzophenone-5-sulphonic acid or its sodium salt (sulisobenzone, sulisobenzone sodium) in an amount of from 0.5 to 5% by weight. Particularly preferred UVA absorbers include butylmethoxydibenzoylmethane commercially available as Parsol 1789^{®} from Givaudan.

Other sunscreen agents for use in the compositions of the invention include water soluble compounds such as 2-phenylbenzimidazole-5-sulphonic acid and its sodium, potassium and triethanolamine salts, conveniently present at concentrations of from 0.5 to 8% by weight.

Physical sunscreens can operate in both the UVA and UVB regions, although as the particle size of the material reduces so does its efficacy in the UVA. Physical sunscreens suitable for use in accordance with the invention include titanium dioxide and zinc oxide, or minerals such as talc, mica, kaolin and bentonite, although titanium dioxide is preferred. Conveniently, these may be present in amounts of from 0.5 to 20% by weight, e.g. up to 10% by weight.

The reflecting properties of physical sunblocks depend to an extent on their particle size. Suitable particle sizes are those in the range of up to 200nm, preferably from 15 to 50nm, more preferably about 30nm. In a preferred embodiment the physical sunblocks can be surface treated with hydrophilic or hydrophobic coatings such as silica, alumina, stearates or silicones. This reduces the tendency of the particles to agglomerate, enabling these to be more effectively dispered throughout the formulation and thereby improving their UV screening efficiency.

Combinations of different sunscreens may be used in the compositions of the invention, e.g. combinations of different chemical and/or physical sunscreens or combinations of UVA and UVB sunscreens. Preferred combinations are those providing protection against both UVA and UVB radiation, e.g. combinations of UVA and UVB sunscreens in a ratio of e.g. from 1:1 to 1:3.

Other components which may be present in the compositions of the invention include those known to provide additional protection to the skin against the effects of oxidant species such as superoxide anion, singlet oxygen and peroxyls formed during photochemical reactions. Such components include vitamins A, C and E and their acid esters, superoxide dismutase (SOD) and reduced glutathione and may be present in either a free or a liposomal form, typically at concentrations of from 0.001 to 10% by weight. Conveniently, these may be present within the same liposome as the glutathione.

In a preferred embodiment of the invention, particular antioxidant/liposome combinations can be formulated to provide the desired level and duration of antioxidant activity. For example, although less effective as a free radical scavenger, SOD has a much longer half life than glutathione. A formulation providing a sustained release of anti-oxidants for a prolonged period may therefore be provided through the use of antioxidants comprising a combination of glutathione liposomes together with free SOD or liposomes containing SOD.

The compositions of the invention preferably further comprise at least one pharmaceutically acceptable carrier. As used herein, "pharmaceutically acceptable" is used to define any agent suitable for topical administration and generally recognised as safe for topical application to the skin. A preferred topical carrier is an emulsion.

The pharmaceutically acceptable carrier typically comprises from 0.1 to 99 % by weight of the compositions of the invention, preferably from 80 to 90 % by weight.

Other components which may be present in the compositions of the invention include oils or esters, emulsifiers, thickening and/or stabilising agents, moisturisers, preservatives and/or chelating agents, antioxidants, fragrance and colourings.

Oils or esters may be present either as carrier materials for the sunscreens and/or the delayed release agents or, alternatively, may be present as emollients to provide an acceptable skin-feel. Examples of oils which may be used include mineral oil, plant oils such as sweet almond oil, avocado oil and jojoba oil, each of which may conveniently be present at concentrations of from 0.5 to 20% by weight. Examples of suitable esters include those formed from a fatty acid and fatty alcohol. Preferred esters include isopropyl myristate and octyl cocoate. Esters may conveniently be present in amounts of from 0.5 to 20% by weight.

Suitable emulsifiers include non-ionic, cationic and anionic emulsifiers known in the art. Non-ionic emulsifiers such as cetyl alcohol, cetearyl alcohol, mono-, di- and triglycerides of fatty acids, ethoxylated fatty alcohols or ethoxylated fatty acids may conveniently be present in an amount of from 0.05 to 10% by weight. Anionic emulsifiers such as sodium, potassium or amine salts of fatty acids, of cetyl phosphates or of an acrylates/alkyl acrylate cross polymer may be present in an amount of from 0.01 to 10% by weight.

Examples of suitable thickening/stabilising agents include the carbomer type anionic water phase thickening, gelling and structuring agents, mineral thickeners such as magnesium aluminium silicate and natural gums such as xanthan gum, which may be present in concentrations of from 0.01 to 10% by weight. Other suitable thickening/stabilising agents include oil phase thickening and structuring agents such as fats and fatty acids, e.g. stearic acid; natural and synthetic waxes and microcrystalline hydrocarbon waxes conveniently present at concentrations of from 0.5 to 20% by weight.

A highly preferred component of the compositions of the invention is at least one moisturiser. A variety of moisturisers may be present, conveniently in an amount of from 0.1 to 20% by weight. Humectant or occlusive moisturising systems which encourage the retention of water within the stratum corneum are preferred. A suitable humectant system may include glycerol, sorbitol or propylene glycol or other constituents equivalent to those found naturally in the skin, such as pyrrolidone carboxylic acid. Suitable occlusive systems include silicones, petrolatum or mineral oil.

Conveniently, the compositions of the invention will include at least one preservative agent or chelating agent designed to control the growth of microorganisms within the product or to control the presence of free metal ions. Examples of suitable substances include potassium sorbate, methyldibromoglutaronitrile, methyl, ethyl, propyl and butyl esters of p-aminobenzoic acid and their metal salts, phenoxyethanol, propylene glycol and disodium edetate, conveniently present in an amount of from 0.005 to 5% by weight.

The compositions in accordance with the invention may further comprise one or more antioxidants, conveniently present in an amount of from 0.005 to 1% by weight, and designed to prevent the oxidative degradation of the product or its constituents. Examples of suitable antioxidants include butylatedhydroxytoluene (BHT) or butylatedhydroxyanisole (BHA).

The compositions in accordance with the invention may be formulated in any conventional form, including oils, emulsions, milks, lotions, creams, mousses, sticks, ointments, gels etc. which may be applied topically to the skin by normal application or by spraying.

For those compositions comprising at least one sunscreen agent, the efficiency of the sunscreen is dependent upon the formulation in which it is contained. To be effective, any sunscreen preparation should form a thin, yet even, protective film on the skin. Preferred formulations for depositing a film on the skin surface are those containing components with good spreading properties, e.g. emulsions. Oil-based products are also particularly suitable for use as sunscreen preparations, although whilst giving an even spread, these tend to deposit much thinner films on the skin. The amount of active sunscreen in an oil-based product may therefore need to be adjusted accordingly to provide the desired SPF.

The compositions in accordance with the invention are of particular use in helping to maintain or restore the natural levels of glutathione in the skin and thus are effective in preventing or reducing the effects of free radicals on the skin, or in treating the conditions resulting from the presence of free radicals in the skin, e.g. in treating inflammatory conditions of the skin caused by exposure to UV radiation. Viewed from a further aspect, the invention thus provides compositions as hereinbefore defined for use in the prevention or treatment of conditions resulting from the presence of free radicals in the skin.

Viewed from a yet further aspect the invention provides the use of compositions as hereinbefore defined in the manufacture of a medicament for use in the prevention or treatment of conditions resulting from the presence of free radicals in the skin.

In another aspect the invention provides a method of cosmetic treatment of the human or non-human mammalian body to combat or prevent conditions associated with the presence of free radicals in the skin, said method comprising application to the skin surface of said body an effective amount of a composition as hereinbefore defined.

The compositions of the invention may be applied to the skin on a daily basis as a moisturiser to protect the skin against the harmful effects of UV radiation. Alternatively, these may be applied prior to or during exposure of the skin to higher levels of UV radiation, e.g. during prolonged periods of sunbathing. Those compositions comprising at least one sunscreen agent are particularly suited to application prior to or during sunbathing, whereas those compositions which do not comprise a sunscreen agent are particularly suited to application to the skin after exposure to UV radiation, e.g. in the form of an after-sun product. For use as a sun-tanning preparation, the compositions preferably contain sunscreens which provide adequate protection against both UVA and UVB radiation.

In a further aspect the invention provides the use of a composition comprising glutathione liposomes and at least one sunscreen agent, optionally together with a delayed release carrier, in the manufacture of a medicament for use in protecting the skin from harmful effects of UV radiation.

The compositions of the invention are applied to the skin in an effective amount, e.g. in an amount equivalent to 2mg of the product per cm² of skin.

Preferred embodiments of the invention will now be described by way of the following non-limiting examples:

### Example 1 - Cream Formulation (F30)

| | % by weight |
|---|---|
| *Phase A* | |
| Octylmethoxycinnamte | 7.5 |
| Butylmethoxydibenzoylmethane | 4.0 |
| Titanium Dioxide | 6.0 |
| Stearic acid | 3.5 |
| Glyceryl Stearate | 1.5 |
| PVP/Eicosene Copolymer | 2.0 |
| | |

| *Phase B* | |
|---|---|
| Carbomer | 1.0 |
| Sorbitol | 3.0 |
| Triethanolamine | 1.0 |
| Disodium Edetate Dihydrate | 0.1 |
| Preservative | qs |
| Water | ad 100 |
| | |

| *Phase C* | |
|---|---|
| Glutathione Liposome | 5.0 |
| (containing 0.1% reduced glutathione) | |
| Fragrance | qs |

The ingredients of Phase A are mixed and heated to 75°C. The ingredients of Phase B are mixed and heated to 75°C. Phase A and Phase B are then mixed until homogeneous and allowed to cool. When the mixture has cooled to below 40°C, the ingredients of Phase C are added and mixed slowly. The resultant mixture is allowed to cool to room temperature.

### Example 2 - Lotion Formulation (F25)

| | % by weight |
|---|---|
| *Phase A* | |
| Methylbenzylidine Camphor | 6.0 |
| Butylmethoxydibenzoylmethane | 4.0 |
| Titanium Dioxide | 6.0 |
| Stearic acid | 2.0 |
| Potassium Cetyl Phosphate | 1.0 |
| PVP/Hexadecane Copolymer | 2.0 |
| | |

| *Phase B* | |
|---|---|
| Triethanolamine | 0.1 |
| Disodium Edetate Dihydrate | 0.1 |
| Preservative | qs |
| Carbomer | 0.1 |
| Sorbitol | 5.0 |
| Water | ad 100 |
| | |

| *Phase C* | |
|---|---|
| Glutathione Liposome | 5.0 |
| (containing 0.1% reduced glutathione) | |
| Fragrance | qs |

The formulation is prepared analogously to that described in Example 1.

### Example 3 - Lotion Formulation (F15)

| | % by weight |
|---|---|
| *Phase A* | |
| Octylmethoxycinnamte | 7.0 |
| Butylmethoxydibenzoylmethane | 4.0 |
| Glyceryl Tricaprylate/caprate | 3.0 |
| Pentaerythritol Tetraisostearate | 3.0 |
| C₁₂-C₁₅ Alcohols Benzoate | 3.0 |
| PEG-20 Stearate | 0.4 |
| Sorbitan Stearate | 1.5 |
| Polysorbate 60 | 1.5 |
| Cetostearyl Alcohol | 0.5 |
| Cyclomethicone | 1.0 |
| Tricontanyl PVP | 2.0 |
| | |

| *Phase B* | |
|---|---|
| Glycerol | 5.0 |
| Disodium Edetate Dihydrate | 0.1 |
| Preservative | qs |
| Water | ad 100 |
| | |

| *Phase C* | |
|---|---|
| Glutathione Liposome | 5.0 |
| (containing 0.1% reduced glutathione) | |
| Fragrance | qs |

The formulation is prepared analogously to that described in Example 1.

### Example 4 - efficacy of glutathione liposome formulation

The depth of glutathione penetration into the skin was determined by comparing two different galenic forms: free glutathione in solution (FG) and glutathione encapsulated in a liposome (LG). Since glutathione is a highly reactive free radical scavenger, its presence even in small quantities can be detected by spectroscopic methods.

### Protocol:

Gluthione preparations are applied to the skin, and tape strippings are taken after some fixed interval of time. The tape strippings are assayed for anti-free radical activity by DPPH (1,1 Diphenyl-2-picrylhydrazyl) at 8.76 x 10⁻³ % (w/v) in methanol) colouration/ discolouration tests (Kim et al. Proc. 17th IFSCC Congress (Yokohama), Vol. 1: 3-14, 1994). The number of strippings necessary to obtain total loss of activity is a measure of the depth of penetration (Clark, J. Soc. Cosmet. Chem. 43: 219-227, 1992; Rougier A et al., J. Pharm. Sci. 76: 451-454, 1987; Thibault et al., Proc. 17th IFSCC Congress (Yokohama), Vol. 1: 245-250, 1992).

### Method:

10 healthy male and female volunteers of 22 to 45 years of age participated in the study. Tape strippings were carried out on the volar forearm of each volunteer with the aid of BLENDERM^{®} adhesive tape (3M Santé).

Light absorption measurements were carried out in a KONTRON UV-Vis spectrometer at 517nm.

### Solutions tested:

Reduced glutathione in water at 0.1% concentration (FG).

Glutathione in liposome at equal concentration (LG).

On the arms of each volunteer, the 3 test sites were determined in a random way: one site for application of water (negative control), one for application of free glutathione (FG) and one for application of liposome encapsulated glutathione (LG). The sites were softly washed with a cloth dipped into ethanol to remove lipids, then allowed to dry and equilibrate for 2 minutes before product application.

5 tape strippings were carried out on the control sites after 2µl/cm² application of distilled water and a light massage. 30 minutes later, the strips were then cut into halves (3.5cm of diameter) onto which was placed a plastic bell of 3cm diameter at the bottom and having a small aperture at the top. Then 1ml of ethanol/water (85/15) solution was introduced into the bell and 500µl of DPPH solution in methanol were added. After 10 minutes the reaction was stopped by inverting the bell and emptying its content into a microcell (1ml). The change in colour was measured on a spectrophotometer at 517nm.

Each test solution was then applied to the designated sites (2µl/cm²) by slight massaging and left to dry for 30 minutes. 5 successive strips were then taken and contacted with the DPPH solutions. Colour changes were recorded as described above.

### Results:

The table below shows the average depth of penetration as revealed by the presence of DPPH reactive substances on the successive strips.

The negative controls indicate the absence of detectable glutathione in untreated skin.

As to the free glutathione preparation (FG), the absence of significant penetration is noted: the product remains concentrated within the first two strips.

The liposome encapsulated glutathione (LG), on the other hand, is carried into the deeper layers of the stratum corneum. Statistical analysis of the 10 values showed the difference to be significant at the 95% confidence level.

**Table 1**

| Tape strips containing residual scavenger activity | | | |
|---|---|---|---|
| Person | Control | FG | LG |
| 1 | 0 | 1 | 2 |
| 2 | 0 | 1 | 2 |
| 3 | 0 | 2 | 2 |
| 4 | 0 | 1 | 2 |
| 5 | 0 | 1 | 3 |
| 6 | 0 | 1 | 3 |
| 7 | 0 | 2 | 1 |
| 8 | 0 | 1 | 1 |
| 9 | 0 | 2 | 1 |
| 10 | 0 | 1 | 2 |
| Mean value | 0 | 1.3 | 1.90 |
| SEM | 0 | 0.48 | 0.74 |

## Claims

1. A composition for topical application comprising glutathione liposomes, together with at least one sunscreen agent and/or a delayed release carrier.

2. A composition as claimed in claim 1 comprising at least one sunscreen agent and a delayed release carrier.

3. A composition as claimed in claim 1 or claim 2 wherein said glutathione liposomes are present in an amount of from 0.5 to 10% by weight, preferably from 1.0 to 5% by weight.

4. A composition as claimed in any one of claims 1 to 3 wherein said glutathione liposomes comprise an aqueous solution comprising reduced glutathione enclosed within a multilamellar liposomal matrix.

5. A composition as claimed in claim 4 wherein the concentration of reduced glutathione in the liposomal matrix is from 0.01 to 1.0% by weight, preferably 0.1% by weight.

6. A composition as claimed in claim 4 or claim 5 wherein said liposomal matrix comprises palmitoylcarnitine.

7. A composition as claimed in any preceding claim wherein said delayed release carrier is present in an amount of from 0.5 to 20% by weight, preferably from 1.0 to 10% by weight.

8. A composition as claimed in any preceding claim wherein said delayed release carrier is selected from dimethicone, cetyl methicone, dimethiconol, trimethylsiloxysilicate, phenyltrimethicone, stearoxytrimethylsilane and copolymers thereof, beeswax, candililla wax, paraffin wax, hydrogenated vegetable oils, modified acrylate copolymers, copolymers of polyvinylpyrrolidone (PVP) and mixtures thereof.

9. A composition as claimed in any preceding claim wherein said sunscreen agent is present in an amount of from 0.5 to 30% by weight, preferably from 10 to 20% by weight.

10. A composition as claimed in any preceding claim wherein said sunscreen agent comprises a physical or chemical sunblock or a mixture thereof.

11. A composition as claimed in claim 10 wherein said chemical sunblock is selected from 4-aminobenzoic acid (PABA), 2-ethylhexyl-4-dimethylaminobenzoate (octyldimethyl PABA), 2-ethylhexyl-4-methoxycinnamate (octylmethoxycinnamate), isopentyl-4-methoxycinnamate, 2-ethylhexylsalicylate (octylsalicylate), homomenthylsalicylate (homosalate), 3-(4'-methylbenzylidene)-d-1-camphor, 1-(4-t-butylphenyl)-3-(4-methoxyphenyl)propane-1,3-dione (butylmethoxydibenzoylmethane), 2-hydroxy-4-methoxybenzophenone (oxybenzone, benzophenone-3), 2-hydroxy-4-methoxybenzophenone-5-sulphonic acid and its sodium salt (sulisobenzone, sulisobenzone sodium), 2-phenylbenzimidazole-5-sulphonic acid and its sodium, potassium and triethanolamine salts, and mixtures thereof.

12. A composition as claimed in claim 10 wherein said chemical sunblock is selected from methylbenzylidene camphor, octylmethoxycinnamate, butylmethoxydibenzoylmethane and mixtures thereof.

13. A composition as claimed in any one of claims 10 to 12 wherein said physical sunblock is selected from titanium dioxide, zinc oxide, talc, mica, kaolin, bentonite and mixtures thereof.

14. A composition as claimed in any preceding claim further comprising one or more compounds selected from vitamins A, C and E and their acid esters, superoxide dismutase and reduced glutathione, each of which may be present in a free or a liposomal form.

15. A composition as claimed in any preceding claim for use in the prevention or treatment of conditions resulting from the presence of free radicals in the human or non-human mammalian skin.

16. Use of a composition as claimed in any one of claims 1 to 14 in the manufacture of a medicament for use in the prevention or treatment of conditions resulting from the presence of free radicals in the human or non-human mammalian skin.

17. A method of cosmetic treatment of the human or non-human mammalian body to combat or prevent conditions associated with the presence of free radicals in the skin, said method comprising application to the skin surface of said body an effective amount of a composition as claimed in any one of claims 1 to 14.

18. A composition comprising glutathione liposomes and at least one sunscreen agent, optionally together with a delayed release carrier, for use in protecting the skin of a human or non-human mammalian body from the harmful effects of UV radiation.

19. Use of a composition as defined in claim 18 in the manufacture of a medicament for use in protecting the skin of a human or non-human mammalian body from the harmful effects of UV radiation.
